# FASCICULE DE BREVET EUROPEEN

(11) **EP 3 746 185 B1**
(45) Date de publication et mention de la délivrance du brevet: **29.03.2023**
(21) Numéro de dépôt: 19705105.5
(22) Date de dépôt: 30.01.2019
(51) Int. Cl.: A61Q 19/08, A61K 8/9728, A61Q 17/00, A61K 8/99

(54) **EXTRAIT DE METSCHNIKOWIA REUKAUFII ET UTILISATION EN COSMETIQUE**
METSCHNIKOWIA-REUKAUFII-EXTRAKT UND VERWENDUNG IN DER KOSMETIK
METSCHNIKOWIA REUKAUFII EXTRACT AND USE IN COSMETICS

(30) Priorité: 31.01.2018 FR 1870099
(43) Date de publication de la demande: 09.12.2020
(73) Titulaire: Societe Industrielle Limousine d'Application Biologique, 19130 Objat (FR)
(72) Inventeur: PAUFIQUE, Jean, 19130 OBJAT (FR)
(74) Mandataire: Aquinov
(86) Numéro de dépôt international: PCT/EP2019/052252
(87) Numéro de publication internationale: WO 2019/149754

(56) Documents cités:
- EP-A1- 1 065 276
- EP-A1- 2 727 579
- WO-A1-2017/195870
- FR-A1- 3 008 891
- IRNAYULI R. SITEPU ET AL: "Oleaginous yeasts for biodiesel: Current and future trends in biology and production", BIOTECHNOLOGY ADVANCES., vol. 32, no. 7, 1 novembre 2014 (2014-11-01), pages 1336-1360, XP055251520, GB ISSN: 0734-9750, DOI: 10.1016/j.biotechadv.2014.08.003
- Sezai Türkel ET AL: "isolation and characterization of new Metschnikowia pulcherrima strains as Producers of the antimicrobial pigment pulcherrimin", , 8 janvier 2009 (2009-01-08), pages 405-410, XP055252106, Extrait de l'Internet: URL:http://www.degruyter.com/dg/viewarticl e.fullcontentlink:pdfeventlink/$002fj$002f znc.2009.64.issue-5-6$002fznc-2009-5-618$0 02fznc-2009-5-618.pdf?t:ac=j$002fznc.2009. 64.issue-5-6$002fznc-2009-5-618$002fznc-20 09-5-618.xml [extrait le 2016-02-22]

## Description

La présente invention concerne un extrait d'une levure spécifique et son utilisation en cosmétique, en particulier pour sa capacité à agir sur le microbiote cutané et notamment sur le microbiote des peaux matures.

La peau cohabite avec de nombreuses communautés bactériennes. Les études visant à analyser les interactions entre la peau et son microbiote se sont multipliées au cours des dernières années et les consommateurs sont informés et éduqués sur les bienfaits des bactéries et l'importance de préserver cette flore. En effet, un microbiote cutané régulé à la surface de la peau conditionne l'immunité, la nutrition et la défense contre les agressions pathogènes. Ainsi, cette symbiose entre la peau et son microbiote est bénéfique et essentielle à sa beauté. Malheureusement, dans certaines conditions, cet équilibre fragile peut être rompu et conduit à l'apparition de désordres cosmétiques au niveau de la peau. C'est le cas en particulier lors du vieillissement cutané qui entraîne un déséquilibre de la flore de la peau responsable de la détérioration de la qualité de la peau et en particulier d'un teint terne. L'objectif de l'invention est de proposer une solution capable d'agir sur le microbiote cutané, en particulier des peaux matures, pour le rééquilibrer et ainsi améliorer la qualité de la peau notamment l'éclat du teint.

Pour y répondre, l'invention vise l'utilisation d'une levure particulière, *Metschnikowia reukaufii.*

*Metschnikowia reukaufii* est l'espèce dominante et spécialiste des nectars floraux. Dotée d'un métabolisme remarquable, elle a développé des propriétés d'adaptation exceptionnelles à ce substrat très sélectif et éphémère. Elle peut se trouver dans le nectar de différentes plantes, et en particulier dans celui de la plante emblématique, raffinée et très nectarifère : *Hoya Carnosa.* Cette plante, également appelée fleur de porcelaine en raison de ses fleurs lumineuses, est d'un aspect vernissé et d'un nacré incroyable, et donne l'impression d'une fleur façonnée de manière merveilleuse. Dans le monde des Hoyas, monde extrêmement riche, cette plante a privilégié l'atout nectar pour assurer son attractivité. La fleur de porcelaine délivre ainsi chaque jour une véritable pluie de nectar habité par des microorganismes qui lui assure protection et régénération.

*Metschnikowia reukaufii* n'a jamais été utilisée en cosmétique, et de façon étonnante, selon l'invention, elle permet d'améliorer la qualité de la peau, en particulier l'éclat du teint, notamment en agissant sur l'équilibre du microbiote cutané.

En effet, seule l'utilisation d'hydrolysats de *Metschnikowia agaves* pour un effet anti-rides (EP2727579) ou un effet sur l'éclat du teint (FR30008891) ont été décrit. La demande de brevet WO2017/195870 décrit un extrait de levure pouvant être un extrait de *Metschnikowia reukaufii* parmi cinq autres levures, sans préciser la nature de l'extrait.

Enfin, Türkel et al. : « Isolation and characterization of new Metschnikowia pulcherrima strains as producers of the antimicrobial pigment pulcherrimin », 8 janvier 2009. p. 405-410, nous enseigne que d'autres souches de *Metschnikowia reukaufii* présentent des propriétés antibactériennes, ce qui n'est pas satisfaisant pour maintenir le microbiote de la peau.

Ainsi, un objet de l'invention est par conséquent un principe actif cosmétique comprenant au moins un extrait, en l'espèce un hydrolysat de *Metschnikowia reukaufii,* ainsi que l'utilisation de ce principe actif ou d'une composition en contenant pour un traitement cosmétique en application topique sur la peau.

L'invention vise également les compositions cosmétiques comprenant au moins un hydrolysat de *Metschnikowia reukaufii,* ainsi qu'un procédé cosmétique de traitement de la peau pour un effet d'amélioration et/ou de sublimation de l'éclat du teint, qui consiste en l'application topique sur la peau d'une telle composition.

D'autres caractéristiques et avantages ressortiront de la description détaillée de l'invention qui va suivre, en regard des figures annexées sur lesquelles :
- La Figure 1 représente la répartition par phytum des bactéries présentes sur les peaux jeunes et âgées avant et après 28 jours de traitement avec un principe actif selon l'invention, et
- La Figure 2 représente la répartition par genre des bactéries présentes sur les peaux jeunes et âgées avant et après 28 jours de traitement avec un principe actif selon l'invention.

### DEFINITIONS

Par « actif cosmétique » ou « principe actif cosmétique » au sens de l'invention, on entend au moins une molécule, préférentiellement un ensemble de molécules présentant un effet cosmétique sur la peau, en particulier sur les cellules cutanées. Il ne s'agit pas d'un actif permettant de stabiliser une composition.

Par « *Metschnikowia reukaufii* » au sens de l'invention, on entend toute levure portant cette classification, de la famille des Metschnikowiacées, qu'elle soit issue de fleur de porcelaine ou d'une autre plante. Elle peut être aussi référencée sous les appellations synonymes, *Metschnikowia zygota, Nectaromyces cruciatus, Nectaromyces reukaufii, Anthomyces reukaufii.* Ce terme exclut les levures génétiquement modifiées.

Par « extrait de *Metschnikowia reukaufii »* au sens de l'invention, on entend tout extrait issu de la levure *Metschnikowia reukaufii,* obtenu par un procédé d'extraction des molécules natives de la levure ou de transformation (par exemple par hydrolyse) des molécules natives de la levure. On entend également le surnageant issu de la culture de la levure, toute molécule excrétée par la levure, toute molécule de la paroi cellulaire de la levure. En revanche le terme « extrait de *Metschnikowia reukaufii* » au sens de l'invention exclut les milieux de cultures de la levure.

Par « fraction protéique » ou « composés peptidiques » de l'hydrolysat de *Metschnikowia reukaufii* au sens de l'invention on entend l'ensemble des protéines et/ou peptides présents dans l'extrait de *Metschnikowia reukaufii.*

Par « hydrolysat de *Metschnikowia reukaufii*» au sens de l'invention, on entend tout extrait issu de la levure *Metschnikowia reukaufii,* obtenu par un procédé comprenant au moins une étape d'hydrolyse enzymatique ou chimique.

Par « peaux matures » on entend des peaux d'un âge supérieur ou égal à 45 ans.

Par « support d'atomisation » au sens de l'invention on entend un adjuvant neutre pouvant être ajouté dans une solution lors du séchage par atomisation.

### DESCRIPTION DETAILLEE DE L'INVENTION

L'invention se rapporte donc à un principe actif cosmétique comprenant au moins un extrait, en l'espèce un hydrolysat de *Metschnikowia reukaufii.*

De façon préférée, l'extrait de *Metschnikowia reukaufii* comprend au moins des peptides. Préférentiellement, l'extrait de *Metschnikowia reukaufii* comprend des peptides ayant un poids moléculaire inférieur à 5000 Da, préférentiellement inférieur à 3500 Da, très préférentiellement inférieur à 2000 Da. Ces peptides jouent un rôle important dans l'efficacité du principe actif selon l'invention, en particulier sur l'expression de HBD3 marqueur de la fonction barrière immune de la peau.

De façon préférée, les peptides ayant un poids moléculaire inférieur à 3500 Da représentent au moins 90% en poids de la fraction protéique de l'extrait, encore plus préférentiellement les peptides ayant un poids moléculaire inférieur à 2000Da représentent au moins 60% en poids de la fraction protéique de l'extrait.

Dans l'extrait, la fraction protéique représente au moins 20% en poids de matière sèche de l'extrait, préférentiellement entre 20 et 60%.

La teneur en composés peptidiques dans les extraits selon l'invention est déterminée par la méthode de LOWRY (Lowry et al., Protein measurement with the folin reagent, J. Biol. Chem., 193, 265, 1951) ou par le dosage de l'azote total selon la méthode de KJELDAHL (référence : Official method of analysis of the A.O.C., 12th ed. W Horwitz, E.D., New-York, 15-60, 1975). Préférentiellement elle est déterminée selon la méthode de KJELDAHL.

L'extrait selon l'invention peut également comprendre d'autres constituants, notamment des sucres et/ou des minéraux.

Préférentiellement, les sucres, lorsqu'ils sont présents, représentent moins de 30% en poids de matière sèche de l'extrait. De façon préférée les sucres présents dans l'extrait selon l'invention sont des oligosaccharides et des polysaccharides de masses molaires comprises entre 180 et 9000 Da (soit un degré de polymérisation supérieur à 1 et inférieur à 50), préférentiellement de masses molaires comprises entre 180 et 2520 Da, (soit un degré de polymérisation supérieur à 1 et inférieur à 14). Préférentiellement les sucres comprennent du glucose et du mannose sous forme d'oligosaccharides avec une masse molaire moyenne de 557 Da.

La teneur en sucres dans l'extrait peut être déterminée par la méthode de DUBOIS (Dubois M. et al., Analytical chemistry, 28, 3, 350-356, 1956).

Les minéraux, lorsqu'ils sont présents dans l'extrait, sont préférentiellement notamment le potassium, le sodium, le chlore, le phosphore. L'analyse des minéraux constituant les cendres de l'extrait selon l'invention peut être réalisée par spectrométrie d'émission optique (ICP/OES) et le dosage des ions chlorures par titration au nitrate d'argent.

Préférentiellement, le taux de cendres est compris entre 20 et 60% en poids de matière sèche de l'extrait, encore plus préférentiellement compris entre 25 et 45%.

La teneur en cendres brutes peut être déterminée par la pesée des résidus issus de l'incinération des échantillons de l'extrait selon l'invention à 550°C dans un four à moufle électrique.

L'extrait selon l'invention peut être obtenu par tout type de procédé d'extraction ou de transformation des molécules natives de la levure permettant d'obtenir un hydrolysat, c'est-à-dire qu'il est obtenu par hydrolyse de la levure. Il peut être obtenu par une hydrolyse chimique (acide ou basique) ou enzymatique. Préférentiellement, l'extrait selon l'invention est un hydrolysat enzymatique. Préférentiellement, l'extrait est un extrait aqueux.

Le principe actif selon l'invention peut se présenter sous forme solide ou sous forme liquide. Lorsqu'il se présente sous forme liquide, le principe actif selon l'invention est préférentiellement constitué par l'extrait tel que décrit précédemment. Il se présente généralement sous forme d'un liquide limpide, avec une faible odeur et une couleur jaune. Il peut toutefois être décoloré par tout procédé connu de l'homme du métier.

Lorsqu'il se présente sous forme solide le principe actif selon l'invention est préférentiellement constitué par l'extrait tel que précédemment décrit et par un support choisi parmi la maltodextrine, la gomme arabique, la lécithine de soja ou l'isomalt. Selon un mode de réalisation, particulièrement adapté, l'extrait représente au moins 25% en poids du principe actif.

L'extrait constituant le principe actif selon l'invention peut être obtenu par tout procédé d'extraction. Préférentiellement, il est obtenu par un procédé comprenant une étape d'hydrolyse enzymatique, en particulier au moins une étape d'hydrolyse enzymatique des protéines c'est-à-dire une hydrolyse réalisée par voie enzymatique au moyen d'enzymes protéolytiques. Il peut s'agir par exemple de protéases d'origine végétale ou issues de microorganismes.

Préalablement au procédé d'obtention de l'extrait en tant que tel, il convient de produire la biomasse de *Metschnikowia reukaufii.* Cette étape est réalisée selon le mode de culture des levures dans un milieu adapté à leur développement, de manière classique pour l'homme de métier. Avant culture, il est possible de prélever la levure et de l'isoler, préférentiellement à partir de nectar de fleurs, en particulier à partir de nectar de la fleur de porcelaine.

Une fois la biomasse obtenue, on réalise une extraction en vue d'obtenir des molécules actives, préférentiellement cette extraction peut contenir au moins une hydrolyse. Selon un mode de réalisation particulièrement adapté, le principe actif est obtenu par la mise en oeuvre des étapes suivantes :
- solubilisation de levures *Metschnikowia rekauffi* dans l'eau, préférentiellement à raison d'au moins 50g/l,
- séparation des phases soluble et insoluble par décantation, pour récupérer la phase soluble,
- filtrations,
- filtration stérilisante.

Une étape d'hydrolyse, préférentiellement une hydrolyse enzymatique, et encore plus préférentiellement au moyen d'une protéase peut être envisagée dans le procédé de production du principe actif selon l'invention. Cette étape est préférentiellement réalisée après solubilisation et avant séparation des phases.

Le procédé peut éventuellement comprendre une étape de traitement thermique de l'extrait, en particulier pour éliminer les activités enzymatiques résiduelles. Cette étape est préférentiellement réalisée avant l'étape de séparation des phases, et lorsqu'il y a une étape d'hydrolyse, après hydrolyse.

Des étapes de décoloration ou désodorisation peuvent aussi être ajoutées.

L'extrait peut ensuite éventuellement être associé à un support et séché, pour se présenter sous forme solide.

Les étapes des procédés décrits ci-avant, prises individuellement, sont usuelles dans le domaine des extractions d'actifs à partir de matières premières naturelles et l'homme du métier est à même d'en ajuster les paramètres réactionnels sur la base de ses connaissances générales.

Le principe actif selon l'invention est particulièrement efficace pour un traitement cosmétique, non thérapeutique.

L'invention a par conséquent également pour objet l'utilisation d'un principe actif cosmétique tel que décrit précédemment ou d'une composition en contenant pour un traitement cosmétique en application topique sur la peau, en particulier pour améliorer la qualité de la peau et notamment pour sublimer l'éclat du teint. La qualité de la peau peut se définir notamment sur trois critères :
- une fonction barrière mécanique intacte,
- et une fonction barrière immune non altérée,
- et un équilibre du microbiote cutané.

Une peau de bonne qualité présente un éclat du teint sublimé.

L'utilisation selon l'invention est préférentiellement destinée aux peaux matures.

Le principe actif selon l'invention peut en particulier être utilisé :
- pour réguler l'écologie cutanée, et notamment pour rééquilibrer le microbiote, préférentiellement des peaux matures, en particulier :
   o pour réduire la diversité bactérienne, et/ou
   o pour diminuer l'abondance du phylum *Proteobacteria* et du genre *Corynebacterium,*
- et/ou pour renforcer la barrière mécanique de la peau, en particulier pour stimuler la production des peptides antimicrobiens de la peau,
- et/ou pour renforcer la barrière immune de la peau, en particulier pour stimuler la synthèse de la cohésion et de la différenciation des cellules cutanées et/ou pour réduire les pertes insensibles en eau de la peau.

La peau est un organe barrière complexe qui héberge à sa surface de nombreux microorganismes (bactéries, champignons, virus). Cette microflore colonisant le *stratum corneum* constitue le microbiote cutané. Il est acquis à la naissance et évolue pour atteindre un état d'équilibre à l'âge adulte. Sa composition et son abondance sont influencées par la génétique et le mode de vie. Ceci garantit le caractère unique du microbiote entre les individus qui peut ainsi être qualifié d'empreinte microbiologique. Les microorganismes et leur hôte vivent en parfaite harmonie. Un microbiote équilibré est sans danger et bénéfique pour son hôte. Parmi la diversité de la flore cutanée, quatre groupes principaux de bactéries (ou *phyla*) ont été caractérisés : *Actinobacteria, Firmicutes, Proteobacteria, Bacteroidetes* avec trois espèces prédominantes : *Corynebacteria, Propionibacteria* et *Staphylococci.*

Le microbiote participe au développement et au maintien de l'équilibre de son hôte. Les microorganismes et la barrière cutanée agissent en synergie pour protéger la peau des agressions extérieures. La flore cutanée joue un rôle fondamental puisqu'elle confère à la peau immunité et protection face aux colonisateurs pathogènes potentiels. Le microbiote cutané est aujourd'hui considéré comme une véritable unité fonctionnelle de la peau qui participe à sa santé et à sa beauté.

Cependant, cet équilibre est constamment menacé par divers facteurs, intrinsèques et extrinsèques, pouvant altérer la composition du microbiote et la fonction barrière de la peau. Ce déséquilibre du microbiote, ou dysbiose, perturbe les coopérations microbe-microbe et microbe-hôte et peut conduire à des désordres cutanés. Il est donc primordial de maintenir l'équilibre de l'écosystème cutané pour arborer une peau de qualité exceptionnelle.

Afin d'étudier la relation entre vieillissement et microbiote cutané, les demandeurs ont comparé par méta-séquençage les microbiomes de peaux caucasiennes jeunes et âgées (voir la partie évaluation de l'effet du principe actif selon l'invention). Cette modélisation inédite a mis en évidence chez des volontaires avec une peau mature:
- une diversité bactérienne plus importante ;
- une diminution significative du phylum *Actinobacteria* au profit des *Proteobacteria ;*
- une augmentation significative du genre *Corynebacterium.*

Avantageusement, le principe actif selon l'invention est capable d'agir sur le microbiote cutané, et en particulier de réduire l'abondance :
- de l'embranchement *Proteobacteria,* et
- du genre *Corynebacterium.*

Ainsi, le principe actif selon l'invention est capable de restaurer l'équilibre du microbiote des peaux matures pour l'obtention d'un profil de peaux jeunes.

Par ailleurs, la peau et son microbiote agissent de concert pour prévenir la prolifération de microorganismes pathogènes.

D'une part, l'épiderme participe activement à la défense de la peau *via* l'établissement de deux barrières :
- une barrière mécanique, qui, de par son étanchéité, empêche efficacement les germes de pénétrer. Ce caractère imperméable de la barrière repose sur l'établissement de jonctions serrées et d'une enveloppe cornée rigide qui résulte d'une différenciation kératinocytaire optimale ;
- une barrière immune, qui fait intervenir un système de défenses innées. Les kératinocytes possèdent à leur surface des senseurs du danger qui leur confèrent la capacité à détecter les microorganismes indésirables. Cette fonction est assurée par des protéines de la famille des TLR (Toll-Like Receptors). En réponse à une menace, les kératinocytes synthétisent des peptides antimicrobiens qui neutralisent et éliminent les pathogènes. Parmi ces molécules clés de l'immunité innée, nous retrouvons les β-défensines (hBDs) et les ribonucléases (RNases). La RNase 7 est notamment décrite pour réguler les bactéries du genre *Corynebacterium.*

D'autre part, les « bonnes bactéries » du microbiote amplifient les défenses immunitaires de leur hôte en stimulant la production de peptides antimicrobiens par les kératinocytes. Ceci démontre la collaboration synergique existant entre l'hôte et son microbiote. De cette synergie dépend l'intégrité de la peau.

Cependant, avec l'âge, les défenses innées sont affaiblies. La fragilité aux invasions pathogènes est augmentée et une altération de la fonction barrière apparaît. Avantageusement, le principe actif selon l'invention est capable d'agir sur les barrières mécanique et immune.

En particulier, le principe actif selon l'invention augmente la synthèse de trois protéines marqueurs de la fonctionnalité de la barrière mécanique de la peau : claudine-1, filaggrine, loricrine, et permet également de diminuer les pertes insensibles en eau ce qui est caractéristique d'une amélioration de la fonction barrière mécanique de la peau.

De plus, le principe actif selon l'invention renforce également l'efficacité de la barrière immune *in vitro.* L'actif selon l'invention augmente également la synthèse du senseur du danger TLR2, des peptides antimicrobiens β-défensine 2 (hBD2), β-défensine 3 (hBD3) et de la riboNucléase 7 (RNase 7), ce qui montre qu'il est capable de restaurer au cours du vieillissement l'immunité innée de la peau, un élément essentiel à l'équilibre du microbiote. En restaurant la fonctionnalité des barrières immune et mécanique de la peau, particulièrement affaiblies avec l'âge, le principe actif selon l'invention rééquilibre le microbiote des peaux matures. Ses actions rééquilibrante et protectrice conduisent à l'amélioration de la qualité de la peau.

En effet, il sublime l'éclat du teint en augmentant le rayonnement de la peau et la couleur rose. La peau est nourrie durablement, plus lumineuse avec moins d'imperfections.

Le principe actif selon l'invention est préférentiellement utilisé dans des compositions, ces compositions comprenant un milieu cosmétiquement acceptable. Il s'agit de compositions dans différentes formes galéniques, adaptées à une application par voie topique sur la peau. Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux ou poudres. Elles peuvent être plus ou moins fluides et avoir l'aspect de crèmes ou gels ou tous autres aspects des cosmétiques de soin peau.

Il peut s'agir de compositions comprenant au moins 0,5% d'un extrait de *Metschnikowia reukauffi* selon la présente invention, préférentiellement entre 0,5 et 10%.

Ces compositions comprennent, outre l'actif, un milieu physiologiquement acceptable et de préférence cosmétiquement acceptable, c'est-à-dire qui ne provoque pas de sensations d'inconfort inacceptables pour l'utilisateur telles que des rougeurs, tiraillements ou picotements.

Les compositions selon l'invention peuvent contenir comme adjuvant au moins un composé choisi parmi :
- les huiles, qui peuvent être choisies notamment parmi les huiles de silicone, linéaires ou cycliques, volatiles ou non volatiles ;
- les cires, telles que l'ozokérite, la cire de polyéthylène, la cire d'abeille ou la cire de carnauba,
- les élastomères de silicone,
- les tensioactifs, de préférence émulsionnants, qu'ils soient non ioniques, anioniques, cationiques ou amphotères,
- les co-tensioactifs, tels que les alcools gras linéaires,
- les épaississants et/ou gélifiants,
- les humectants, tels que les polyols comme la glycérine,
- les colorants, les conservateurs, les charges,
- les tenseurs,
- les séquestrants,
- les parfums,
- et leurs mélanges, sans que cette liste soit limitative.

Des exemples de tels adjuvants sont cités notamment dans le Dictionnaire CTFA (*International Cosmetic Ingrédient Dictionary and Handbook* publié par le *Personal Care Product Council*). Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Ces compositions sont notamment destinées à être utilisées pour améliorer la qualité de la peau, en particulier des peaux matures, en agissant sur la fonctionnalité des barrières mécanique et/ou immune de la peau et sur l'équilibre du microbiote cutané.

L'invention vise ainsi également un procédé non thérapeutique, à savoir un procédé cosmétique non thérapeutique de traitement de la peau pour un effet d'amélioration et/ou de sublimation de l'éclat du teint, qui consiste en l'application topique sur la peau d'une composition comprenant un principe actif selon l'invention, en particulier sur les peaux matures, et spécifiquement pour rééquilibrer le microbiote des peaux matures. Préférentiellement la composition comprenant le principe actif cosmétique selon l'invention est appliquée au moins une fois par jour pendant au moins 15 jours. De façon préférée, la composition est une composition selon l'invention.

Afin d'illustrer ces effets cosmétiques sur la qualité de la peau, en particulier sur l'éclat du teint, les fonctions barrières mécanique et immune et le microbiote cutané, les exemples suivants ainsi que des résultats d'essais sont présentés en suivant.

### EXEMPLES

### Exemple 1 : Principe actif selon l'invention

Le principe actif selon l'exemple 1 est obtenu par la mise en oeuvre des étapes suivantes :
- solubilisation de la biomasse de *Metschnikowia rekaufii* dans l'eau à raison de 100g/l,
- une hydrolyse enzymatique au moyen d'une protéase,
- traitement thermique de l'hydrolysat,
- séparation des phases soluble et insoluble par décantation, pour récupérer la phase soluble,
- filtrations, à l'aide d'un filtre permettant d'éliminer les molécules ayant un poids moléculaire supérieur à 5000Da,
- filtration stérilisante sur 0.22µm.

L'hydrolysat obtenu se présente sous forme d'un liquide limpide de couleur jaune.

Il est constitué par :
- Une teneur en matières sèches de 33.5g/l,
- 52% de peptides en poids de matière sèche, (déterminée par la méthode de KJELDAHL), dont 96% des peptides présentent un poids moléculaire inférieur ou égal à 2000Da,
- 10% de sucres en poids de matière sèche, (déterminée par la méthode de DUBOIS), dont la totalité sont des oligosaccharides ayant un poids moléculaire inférieur ou égal à 2500 Da,
- 38% de cendres en poids de matière sèche, (déterminée par la pesée des résidus issus de l'incinération des échantillons de l'hydrolysat à 550°C dans un four à moufle électrique).

Ce principe actif selon l'invention est une solution aqueuse de couleur jaune très clair.

### Exemple 2: Principe actif de référence (hors invention)

Le principe actif selon l'exemple 2 est obtenu par la mise en oeuvre des étapes suivantes :
- solubilisation de la biomasse de *Metschnikowia rekaufii* dans l'eau à raison de 100g/l,
- traitement thermique,
- séparation des phases soluble et insoluble par décantation, pour récupérer la phase soluble,
- filtrations, à l'aide d'un filtre permettant d'éliminer les molécules ayant un poids moléculaire supérieur à 5000Da,
- filtration stérilisante sur 0.22µm.

Le principe actif selon l'exemple 2 se présente sous forme d'un liquide limpide de couleur jaune.

Il est constitué par :
- Une teneur en matières sèches est de 28.9g/l.
- 56% de peptides en poids de matière sèche, (déterminée par la méthode de KJELDAHL),
- 9% de sucres en poids de matière sèche, (déterminée par la méthode de DUBOIS),
- 17% de cendres en poids de matière sèche, (déterminée par la pesée des résidus issus de l'incinération des échantillons de l'hydrolysat à 550°C dans un four à moufle électrique).

### Exemple 3 : composition selon l'invention sous forme de formule de jour

Un exemple de composition selon l'invention sous forme de formule de jour est constitué par :

| A. | Eau | | qsp 100% |
|---|---|---|---|
| | Conservateur | | 1% |
| | Glycerol | | 2% |
| B. | DUB 1632 | (Stéarinerie Dubois) | 3% |
| | DUB MM | (Stéarinerie Dubois) | 1,5% |
| | DUB STGAE30 | (Stéarinerie Dubois) | 3% |
| | DUB ININ | (Stéarinerie Dubois) | 2% |
| | Sophiderme | (Sophim) | 4% |
| | Easynov | (Seppic) | 4% |
| C | Principe actif selon l'invention | | 2,5% |

Le pH de la composition est de 6,0. Elle se présente sous forme d'une émulsion fluide, blanche, sans odeur, brillante, avec une application aisée, un étalement doux et glissant.

La composition peut être obtenue comme suit :
- Mélanger A. Chauffer au bain-marie à 80°C sous agitation magnétique.
- Mélanger B. Chauffer au bain-marie à 80°C sous agitation magnétique.
- A 80°C, émulsionner A dans B sous rotor stator à 1800 tr/min.
- A 30°C additionner C.
- Laisser sous agitation jusqu'à complet refroidissement.

### Exemple 4 : composition selon l'invention sous forme de formule sérum

Un exemple de composition selon l'invention sous forme de formule sérum est constitué par :

| A. | Eau | | qsp 100% |
|---|---|---|---|
| | Conservateur | | 1% |
| | Propylène Glycol | | 2% |
| B. | Montanov L | (Seppic) | 2% |
| | Lanol 1688 | (Seppic) | 4% |
| | Montanov 14 | (Seppic) | 1% |
| | DUB 5545 | (Stéarinerie Dubois) | 3,5% |
| C. | Satiaxane CX911 | (Cargill) | 0,6% |
| | Principe actif selon l'invention | | 2,5% |

Le pH de la composition est de 6,6. Elle se présente sous forme d'une émulsion liquide, blanche, brillante, sans odeur, avec une préhension légère, étalement frais et glissant. Pénétration très rapide, fini doux et sec, effet évanescent.

La composition peut être obtenue comme suit :
- Mélanger A. Chauffer au bain-marie à 80°C sous agitation magnétique.
- Mélanger B. Chauffer au bain-marie à 80°C sous agitation magnétique.
- A 80°C, émulsionner A dans B sous rotor stator à 2000tr/min.
- A 30°C, ajouter C, dans l'ordre indiqué, sous rotor stator à 1500 tr/min.
- Laisser sous agitation jusqu'à complète homogénéisation.

### Exemple 5 : composition selon l'invention sous forme d'un gel siliconé

Un exemple de composition selon l'invention sous forme d'un gel siliconé est constitué par :

| A. | Eau | | qsp 100% |
|---|---|---|---|
| | Conservateur | | 1% |
| | Glycerol | | 3% |
| B. | KF 6017 | (Shin Etsu) | 3% |
| | KF 6028 | (Shin Etsu) | 2% |
| | Sepimax Zen | (Seppic) | 0,5% |
| | Sensanov WR | (Seppic) | 3% |
| C. | DC 73310 | (Dow Corning) | 3% |
| | Principe actif selon l'invention | | 2,5% |
| | Simulgel EPG | (Seppic) | 0,3% |

La composition a un pH de 5,2.

La composition se présente sous forme d'un gel blanc translucide, brillant, sans odeur, texture semi-épaisse, avec une préhension souple, excellente application effet silicone.

La composition peut être obtenue comme suit :
- Mélanger A. Chauffer au bain-marie à 50°C sous agitation magnétique, en veillant à bien disperser le conservateur.
- Mélanger B, bien homogénéiser sous agitation magnétique.
- Emulsionner A dans B, sous rotor stator à 1000 tr/min, pendant 10 minutes puis à 2200 tr/min, jusqu'à complète homogénéisation.
- Puis ajouter C, dans l'ordre indiqué, toujours sous rotor-stator.
- Après quelques minutes réduire l'agitation à 1000 tr/min.

### Exemple 6 : composition selon l'invention sous forme d'un gel émulsionné fluide

Un exemple de composition selon l'invention sous forme d'un gel émulsionné fluide est constitué par :

| A. | Eau | | qsp 100% |
|---|---|---|---|
| | Conservateur | | 1% |
| B. | DUB PGPR | (Stéarinerie Dubois) | 0,5% |
| | DUB RG AE 30 | (Stéarinerie Dubois) | 1% |
| | DUB INID | (Stéarinerie Dubois) | 0,5% |
| | Montanox 80 | (Seppic) | 0,5% |
| | Sepinov EMT | (Seppic) | 1% |
| | Easynov | (Seppic) | 1,5% |
| C. | DUB Velvet Gum | (Stéarinerie Dubois) | 3% |
| | Principe actif selon l'invention | | **2,5**% |
| | DC 200 | (Dow Corning) | 1% |

Le pH de la composition est de 6,5.

La composition se présente sous forme d'un gel émulsionné blanc, brillant, sans odeur, texture fluide, avec une préhension facile, application douce, étalement glissant.

La composition peut être obtenue comme suit :
- Mélanger A. Chauffer au bain-marie à 80°C sous agitation magnétique.
- Mélanger B. Chauffer au bain-marie à 80°C sous agitation magnétique.
- Emulsionner B dans A, sous rotor stator à 1800 tr/min.
- A froid, ajouter C, dans l'ordre, et agiter sous rotor stator à 2000 tr/min.
- Laisser sous agitation jusqu'à complète homogénéisation.

### Exemples d'extraits d'autres levures (hors invention) et comparaison analytique avec l'extrait de levure selon l'invention

Afin de montrer la spécificité de la levure sélectionnée pour le principe actif selon l'invention, nous avons réalisé le procédé d'extraction identique à celui de l'invention sur deux autres levures :
- *Saccharomyces cerevisiae,* levure très utilisée en cosmétique et en alimentaire,
- *Metschnikowia agaves,* levure du même genre que celle de l'invention.

Les caractéristiques des différents extraits sont présentés dans le Tableau 1.

**Tableau 1**

| | Principe actif selon l'invention issu de *Metschnikowia rekaufii* | | Extrait issu de *Metschnikowia agaves* | | Extrait issu de *Saccharomyces cerevisiae* | |
|---|---|---|---|---|---|---|
| Procédé d'extraction | Solubilisation de levures dans l'eau à raison de 100g/l, Hydrolyse enzymatique au moyen d'une protéase, Traitement thermique pour éliminer les activités enzymatiques, Séparation des phases soluble et insoluble par décantation, Filtrations, Filtration stérilisante | | | | | |
| Aspect | Liquide limpide de couleur jaune | | Liquide limpide de couleur jaune | | Liquide opalescent de couleur jaune | |
| Matières sèches (g/l) | 33.8g/l | - | 24.3 | - | 18.1 | - |
| Sucres totaux (g/l) | 2g/l | 6% | 1.5 | 6% | 1.8 | 10% |
| Protéines totales (g/l) (Méthode du Lowry) | 7.8g/l | 23% | 3.6 | 15% | 5.1 | 28% |
| Protéines totales (g/l) (Méthode de Kjeldahl) | 15.6g/l | 47% | 8.7g/l | 36% | 9.6g/l | 53% |
| Minéraux (g/l) | 9.1g/l | 27% | 7.4 | 30% | 5.2 | 29% |

Les chromatogrammes des fractions carbohydrates des deux essais sur *Metschnikowia agaves* et sur *Saccharomyces cerevisiae* ont été comparés avec le principe actif selon l'invention. Les chromatogrammes sont différents. La nature de la fraction saccharidique des trois essais est différente.

### ESSAIS ET RESULTATS

### Démonstration de la modification du microbiote cutané au cours du vieillissement

Des prélèvements du microbiote cutané ont été réalisés au niveau du visage de volontaires à l'aide d'écouvillons stériles sur une zone de 10cm². Chaque écouvillon a ensuite été congelé et stocké avant l'extraction d'ADN. L'analyse a été réalisée par séquençage 16S. L'ADN génomique bactérien est extrait à partir des prélèvements à l'aide du kit DNeasy Power Lyzer Soil (Qigen).

L'analyse de la diversité des bactéries de la peau du visage a été réalisée sur 34 volontaires sains, caucasiens, 17 volontaires jeunes d'âge moyen 28 ± 3 ans, et 17 volontaires âgés d'âge moyen 62 ± 5 ans.

La diversité des bactéries prélevées sur la peau permet de donner l'indice de Shannon qui prend en compte le nombre d'espèces de bactéries présentes et leur abondance relative. Cet indice donne une information sur la structure de l'échantillon.

L'analyse de la taxonomie des bactéries des échantillons permet de les classer selon 7 niveaux : Domaine, Embranchement ou Phylum, Classe, Ordre, Famille, Genre ou Espèce. Les résultats moyens sur la diversité sont les suivants :

**Tableau 2**

| | Jeunes | Agés |
|---|---|---|
| Diversité / Indice de Shannon | 1.30 ± 0.44 | 1.74 ± 0.35 |

Les donneurs âgés présentent une diversité plus importante que les donneurs jeunes, reflétée par l'augmentation significative de l'indice de Shannon.

En se basant sur la répartition des bactéries au niveau de l'embranchement (phytum), les phytum des bactéries présentes sur la peau se répartissent comme suit :

**Tableau 3**

| Répartition des bactéries / Phytum | Jeunes | Âgés |
|---|---|---|
| *Actinobacteria* | 59,2% | 46,7% |
| *Bacteroidetes* | 1,1% | 2,1% |
| *Firmicutes* | 35,3% | 36,2% |
| *Proteobacteria* | 3,5% | 13,9% |
| *Autres* | 0,9% | 1,0% |

On constate qu'au cours du vieillissement cutané, on observe une augmentation de l'abondance relative de l'embranchement *Proteobacteria,* et une diminution de l'abondance relative de l'embranchement *Actinobacteria.*

Par ailleurs, en se basant sur la répartition des bactéries au niveau du genre, l'abondance relative des genres des bactéries présentes sur la peau se répartit comme suit :

**Tableau 4**

| Répartition des bactéries / genre | Jeunes | Âgés |
|---|---|---|
| *Acinetobacter* | 0,5% | 1,2% |
| *Corynebacterium* | 0,8% | 5,0% |
| *Enhydrobacter* | 0,8% | 3,4% |
| *Propionibacterium* | 56,6% | 40,6% |
| *Staphyloccocus* | 32,2% | 31,5% |
| *Streptococcus* | 1,4% | 1,0% |
| *Autres* | 7,7% | 17,3% |

On constate ainsi qu'au cours du vieillissement cutané, on observe une augmentation de l'abondance relative du genre *Corynebacterium,* et une diminution de l'abondance relative du genre *Propionibacterium.*

L'augmentation de la proportion de *Proteobacteria* et *Corynebacteria* avec l'âge traduit une colonisation facilitée de la peau par ces bactéries opportunistes au cours du vieillissement.

### Démonstration de l'effet du principe actif selon l'invention sur le microbiote cutané

34 volontaires sains caucasiens ont appliqués sur le visage biquotidiennement une formule cosmétique contenant 2.5% du principe actif selon l'invention (exemple 1) et une formule cosmétique placebo en hémi-visage. Les prélèvements du microbiote cutané ont été réalisés avant et après 28 jours de traitement cosmétique.

Les 34 volontaires se répartissent comme suit : 17 volontaires jeunes d'âge moyen 28 ± 3 ans, et 17 volontaires âgés d'âge moyen 62 ± 5 ans.

La diversité des bactéries prélevées sur la peau permet de donner l'indice de Shannon qui prend en compte le nombre d'espèces de bactéries présentes et leur abondance relative. Cet indice donne une information sur la structure de l'échantillon.

L'abondance des différents taxons bactériens est donnée en abondance relative. 7 niveaux de taxonomie sont utilisés dans la nomenclature officielle de classification des espèces bactériennes : Domaine, Embranchement ou Phylum, Classe, Ordre, Famille, Genre ou Espèce. L'analyse taxonomique de cette étude a été réalisée à deux niveaux de la classification : l'Embranchement et le Genre.

4 embranchements : *Actinobacteria, Bacteroidetes, Firmicutes et Proteobacteria* ainsi que 6 genres bactériens : *Acinetobacter, Corynebacterium, Enhydrobacter, Propionibacterium, Staphyloccocus et Streptococcus* sont majoritairement retrouvés dans le microbiote cutané et présentés dans cette étude. Les autres taxons sont regroupés dans une catégorie « autres ». Les résultats moyens sur la diversité sont les suivants :

**Tableau 5**

| | Traitement Principe actif selon l'invention | |
|---|---|---|
| Indice de Shannon | Jeunes | Agés |
| J0 | 1.33 ± 0.44 | 1.70 ± 0.47 |
| J28 | 1.32 ± 0.53 | 1.51 ± 0.40 |
| Variation J28-J0 | -1% non significatif | -11% |

Les donneurs âgés présentent une diversité plus importante que les donneurs jeunes, reflétée par l'augmentation significative de l'index de Shannon.

La formulation placebo ne modifie pas la diversité du microbiote cutané des peaux âgées ou des peaux jeunes.

La formulation contenant le principe actif agit sur la diversité du microbiote cutané des peaux âgées en réduisant significativement de 11% l'indice de Shannon. Par contre, il ne modifie pas la diversité et l'écologie bactérienne des donneurs jeunes.

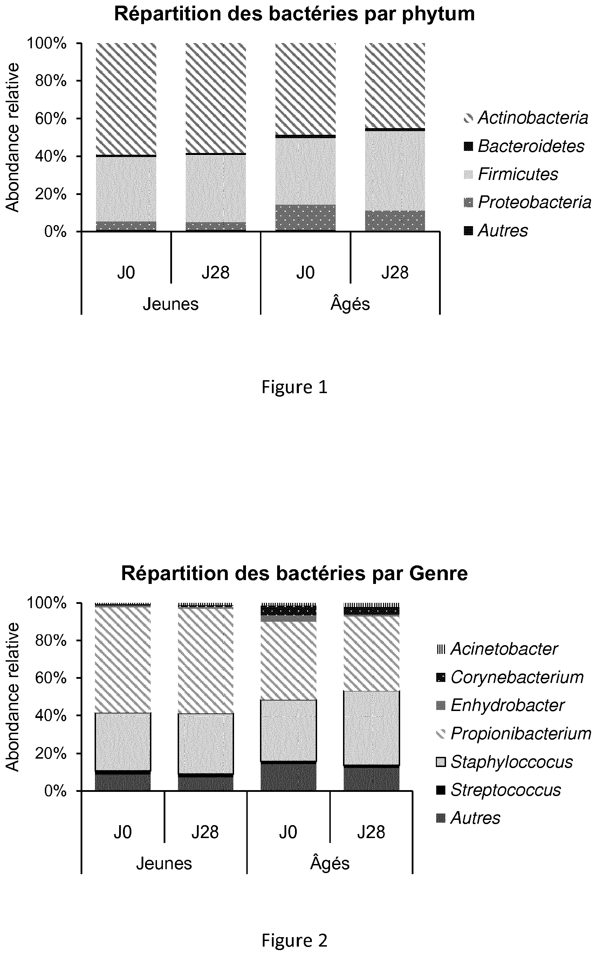

En se basant sur la répartition des bactéries au niveau de l'embranchement (phytum), les phytum des bactéries présentes sur la peau se répartissent comme présenté sur la Figure 1 et dans le Tableau 6.

**Tableau 6**

| | | Traitement Principe actif selon l'invention | |
|---|---|---|---|
| *Répartition des bactéries* / *phytum* | | Jeunes | Âgés |
| *Proteobacteria* | J0 | 4,4% | 13,3% |
| | J28 | 4.1% | 10.6% |
| | Δ(J28-J0) | -7% non significatif | -20% |

Le microbiote des donneurs âgés présente une abondance plus importante de l'embranchement Proteobacteria comparé au microbiote des donneurs jeunes.

Le principe actif selon l'invention agit sur la composition du microbiote cutané en réduisant significativement l'abondance de l'embranchement Proteobacteria de 20%. Il ne modifie pas significativement le microbiote cutané des peaux jeunes.

En se basant sur la répartition des bactéries au niveau du genre, les genres des bactéries présentes sur la peau se répartissent comme présenté sur la Figure 2 et dans le Tableau 7.

**Tableau 7**

| | | Traitement Principe actif selon l'invention | |
|---|---|---|---|
| *Répartition des bactéries* / *phytum* | | Jeunes | Âgés |
| *Corynebacterium* | J0 | 0,7% | 5,2% |
| | J28 | 0.9% | 4.1% |
| | Δ(J28-J0) | +29% | -21% |

Le microbiote des donneurs âgés présente une abondance plus importante du genre *Corynebacterium* comparé au microbiote des donneurs jeunes.

Le principe actif selon l'invention agit sur la composition du microbiote cutané en réduisant significativement l'abondance du genre *Corynebacterium* de 21%. Il ne modifie pas significativement la composition du microbiote des peaux jeunes.

### Démonstration de l'effet d'un extrait de Metschnikowia reukaufii sur la fonction barrière immune

L'influence du principe actif selon l'invention sur la fonction barrière immune a été évaluée sur trois études :
- In vitro sur des cultures de kératinocytes humains, pour déterminer l'expression des peptides anti microbiens HBD2,
- In vitro sur des épidermes reconstruits vieillis (Silabskin^{®} RE vieillis), pour évaluer l'expression des TLR2 et des peptides anti microbiens HBD2, HBD3 et de la protéine anti microbienne RNAse 7,
- In vivo sur 15 volontaires sains, pour déterminer l'expression de la ribonucléase 7 (RNAse 7) protéine antimicrobienne.

Les protocoles opératoires des essais sont décrits en suivants.

### Expression des peptides anti microbiens HBD2

Les kératinocytes humains normaux sont cultivés dans du milieu de culture spécifique et sont traités avec 1% du principe actif selon l'invention ou d'autres extraits de levures (*Metschnikowia agaves, Saccharomyces cerevisiae* tels que présentés dans les exemples). Les ARN totaux sont extraits des kératinocytes après traitement. L'analyse par PCR a permis de déterminer les ARNm de HBD2.

### Expression des TLR2 et des peptides anti microbiens HBD2, HBD3 et de la protéine anti microbienne RNAse 7

Les épidermes reconstruits ont été réalisés au moyen de kératinocytes humains normaux ou vieillis par passages successifs. Les épidermes reconstruits ont été traités par 0.25% ou 0.5% du principe actif selon l'invention. Après 24h de traitement, les épidermes ont été récupérés pour extraire les ARN totaux codant pour HBD2, HBD3, TLR2 ou RNAse 7.

### Expression de la ribonucléase 7 (RNAse 7) protéine antimicrobienne.

Les 15 volontaires ont été traités par une formulation cosmétique contenant 2.5% du principe actif selon l'invention pendant 14 jours d'applications biquotidiennes des avant-bras. Le dosage de RNAse 7 par dosage Elisa a été réalisé suite à une agression mécanique de la peau par stripping.

Les résultats sur l'expression de HBD2 sur des cultures de kératinocytes humains sont présentés dans le Tableau 8 :

**Tableau 5**

| | Expression de HBD2 | Capacité d'induire l'expression de HBD2 |
|---|---|---|
| Témoin | 100% | |
| Traité avec 1% du principe actif selon l'invention de l'exemple 1 | 144% | 44% |
| Traité avec 1% de l'extrait de Metschnikowia agaves | 88% | 0% |
| Traité avec 1% de l'extrait de Saccharomyces cerevisiae | 80% | 0% |

On constate que seul le principe actif selon l'invention stimule l'expression de HBD2 dans les cultures de kératinocytes humains. Les deux extraits de deux autres levures (*Metschnikowia agaves ou Saccharomyces cerevisiae*) ne présente pas la même efficacité. On peut donc conclure que seul l'extrait de la levure sélectionnée *Metschnikowia rekaufii* présente une action sur le microbiote cutané.

Les résultats sur les épidermes reconstruits pour évaluer l'expression des TLR2 et des peptides anti microbiens HBD2, HBD3 et de la protéine anti microbienne RNAse 7 sont les suivants :

**Tableau 9**

| | Expression de TLR2 | Capacité d'induire l'expression de TLR2 |
|---|---|---|
| Epidermes reconstruits normaux | | |
| Témoin | 100% | |

| Epidermes reconstruits vieillis | | |
|---|---|---|
| Témoin | 39% | |
| Traité avec 0.25% du principe actif selon l'invention | 42% | 5% |
| Traité avec 0.5% du principe actif selon l'invention | 62% | 37% |

**Tableau 10**

| | Expression de HBD2 | Capacité d'induire l'expression de HBD2 |
|---|---|---|
| Epidermes reconstruits normaux | | |
| Témoin | 100% | |

| Epidermes reconstruits vieillis | | |
|---|---|---|
| Témoin | 33% | |
| Traité avec 0.25% du principe actif selon l'invention | 50% | 26% |
| Traité avec 0.5% du principe actif selon l'invention | 118% | 127% |

**Tableau 11**

| | Expression de HBD3 | Capacité d'induire l'expression de HBD3 |
|---|---|---|
| Epidermes reconstruits normaux | | |
| Témoin | 100% | |

| Epidermes reconstruits vieillis | | |
|---|---|---|
| Témoin | 47% | |
| Traité avec 0.25% du principe actif selon l'invention | 51% | 8% |
| Traité avec 0.5% du principe actif selon l'invention | 64% | 32% |

**Tableau 12**

| | Expression de RNAse 7 | Capacité d'induire l'expression de RNAse 7 |
|---|---|---|
| Epidermes reconstruits normaux | | |
| Témoin | 100% | |

| Epidermes reconstruits vieillis | | |
|---|---|---|
| Témoin | 60% | |
| Traité avec 0.25% du principe actif selon l'invention | 64% | 9% |
| Traité avec 0.5% du principe actif selon l'invention | 74% | 35% |

Testé à 0.5% le principe actif selon l'invention augmente significativement l'expression des peptides anti-microbiens, comme la beta defensine 2, de la beta defensine 3, de TRL2 et de RNAse7 dans les épidermes reconstruits.

Les résultats sur les volontaires pour déterminer l'expression de la ribonucléase 7 (RNAse 7) sont les suivants :

**Tableau 13**

| | Concentration moyenne en RNAse 7 | Δ / ZNT | Δ / placebo |
|---|---|---|---|
| Zone non traitée | 0.637 | | |
| Placebo | 1.222 | +92% | |
| Principe actif selon l'invention à 2.5% | 1.978 | -211% | +119% |

Après un traitement de 14 jours et en comparaison au placebo, le principe actif selon l'invention formulé à 2.5% en gel émulsionné, favorise le relargage d'un peptide anti-microbien, la RNAse 7, dont le large spectre d'activité contribue au système de défense de la peau.

Le principe actif selon l'invention booste de 119% la sécrétion de RNAse 7. Cet effet est observé chez 87% des volontaires.

Le principe actif selon l'invention booste donc les peptides anti-microbiens de la peau.

### Démonstration de l'effet d'un extrait de Metschnikowia reukaufii sur la fonction barrière mécanique

L'influence du principe actif selon l'invention sur la fonction barrière mécanique a été évaluée sur deux études :
- In vitro sur des épidermes reconstruits vieillis (Silabskin^{®} RE vieillis), pour évaluer l'expression des jonctions serrées : claudine 1 et la différenciation kératinocytaire : filaggrine et loricrine
- In vivo sur 20 volontaires sains caucasiens, pour déterminer la perte insensible en eau,
- In vivo sur 31 volontaires sains asiatiques, pour déterminer la perte insensible en eau.
Les épidermes reconstruits ont été réalisés au moyen de kératinocytes humains normaux ou vieillis par passages successifs. Les épidermes reconstruits ont été traités par 0.25% du principe actif selon l'invention. Après 24h de traitement, les épidermes ont été récupérés pour extraire les ARN totaux codant pour claudine1, filaggrine et loricrine.

Les volontaires ont été traités par une formulation cosmétique contenant 2.5% du principe actif selon l'invention pendant 28 jours d'application biquotidienne des avant-bras. La perte insensible en eau a été mesurée au moyen d'un Tewamètre.

Les résultats sur les épidermes reconstruits pour évaluer l'expression des jonctions serrées : claudine 1 et sur la différenciation kératinocytaire : filaggrine et loricrine, sont les suivants :

**Tableau 14**

| | Expression de Claudine 1 (UA) | Capacité d'induire l'expression de Claudine 1 |
|---|---|---|
| Epidermes reconstruits normaux | | |
| Témoin | 60.7 | |

| Epidermes reconstruits vieillis | | |
|---|---|---|
| Témoin | 48.6 | |
| Traité avec 0.25% du principe actif selon l'invention (exemple 1) | 61.0 | +103% |

**Tableau 15**

| | Expression de Filaggrine (UA) | Capacité d'induire l'expression de Filaggrine |
|---|---|---|
| Epidermes reconstruits normaux | | |
| Témoin | 27.8 | |

| | | |
|---|---|---|
| Témoin | 22.8 | |
| Traité avec 0.25% du principe actif selon l'invention (exemple 1) | 27.2 | +90% |

**Tableau 16**

| | Expression de Loricrine (UA) | Capacité d'induire l'expression de Loricrine |
|---|---|---|
| Epidermes reconstruits normaux | | |
| Témoin | 29.9 | |

| Epidermes reconstruits vieillis | | |
|---|---|---|
| Témoin | 15.7 | |
| Traité avec 0.25% du principe actif selon l'invention (exemple 1) | 21.1 | +38% |

Testé à 0.25% sur les épidermes reconstruits vieillis, le principe actif selon l'invention augmente significativement la synthèse de claudine 1 de 103%, de filaggrine de 90% et de loricrine de 38%.

L'effet favorable sur la fonction barrière a été confirmé in vivo à la fois sur des peaux caucasiennes ou asiatiques. En comparaison au placebo, le principe actif selon l'invention permet de réduire de 9.1% les pertes insensibles en eau sur le panel caucasien, et de 9.2% sur le panel asiatique.

Le principe actif selon l'invention contribue donc bien à la restauration d'une fonction barrière mécanique fonctionnelle.

### Démonstration de l'effet d'un extrait de Metschnikowia reukaufii sur l'éclat du teint

L'influence du principe actif selon l'invention sur l'éclat du teint sur volontaires a été évaluée sur deux panels :
- Un panel de 20 volontaires caucasiens,
- Un panel de 31 volontaires asiatiques.

Les volontaires ont été traités par une formulation cosmétique contenant 2.5% du principe actif selon l'invention (exemple 1) pendant 28 jours d'application biquotidienne sur le visage. L'éclat du teint est évalué par scorage clinique par les experts.

Sur le panel caucasien, après 28 jours d'application biquotidienne, le principe actif selon l'invention augmente significativement le rayonnement de la peau de 9.5% et la couleur rose de 13.0%. Il permet aussi de réduire significativement la couleur olive de 9.3% et l'état de fatigue des yeux de 8.0%.

Sur le panel asiatique, l'éclat du teint a été évalué par scorage clinique. Après 28 jours d'application biquotidienne, le principe actif selon l'invention augmente la luminosité de la peau de +12.2%, son rayonnement de +9.2% et sa transparence de la peau de +8.2%.

Le principe actif selon l'invention est perçu par les volontaires caucasiens et asiatiques comme plus efficace que le placebo. D'une manière générale, les volontaires jugent leur peau nourrie durablement. Elles trouvent que le principe actif selon l'invention améliore la luminosité de leur peau et limite les imperfections.

## Revendications

1. Principe actif cosmétique comprenant au moins un extrait de *Metschnikowia reukaufii,* **caractérisé en ce que** l'extrait est un hydrolysat de *Metschnikowia reukaufii.*

2. Principe actif cosmétique selon la revendication 1, **caractérisé en ce que** l'extrait de *Metschnikowia reukaufii* comprend au moins des peptides.

3. Principe actif cosmétique selon la précédente revendication, **caractérisé en ce que** les peptides représentent au moins 20% en poids de matière sèche de l'extrait, déterminée par la méthode de KJELDAHL.

4. Principe actif cosmétique selon la précédente revendication, **caractérisé en ce que** les peptides représentent entre 20 et 60% en poids de matière sèche de l'extrait, déterminée par la méthode de KJELDAHL.

5. Principe actif cosmétique selon l'une des précédentes revendications, **caractérisé en ce que** l'extrait de *Metschnikowia reukaufii* comprend des peptides dont au moins 90% ont une masse moléculaire inférieure à 3500 Da.

6. Principe actif cosmétique selon l'une des précédentes revendications, **caractérisé en ce que** l'extrait de *Metschnikowia reukaufii* comprend des peptides dont au moins 60% ont une masse moléculaire inférieure à 2000 Da.

7. Principe actif cosmétique selon l'une des précédentes revendications, **caractérisé en ce que** l'extrait de *Metschnikowia reukaufii* comprend également des sucres.

8. Principe actif cosmétique selon la précédente revendication, **caractérisé en ce que** les sucres comprennent du glucose et du mannose sous forme d'oligosaccharides avec une masse molaire moyenne inférieur à 9000Da.

9. Principe actif cosmétique selon l'une des précédentes revendications, **caractérisé en ce que** l'extrait de *Metschnikowia reukaufii* est un hydrolysat enzymatique de *Metschnikowia reukaufii.*

10. Utilisation d'un principe actif cosmétique selon l'une des revendications 1 à 9 ou d'une composition en contenant pour un traitement cosmétique en application topique sur la peau.

11. Utilisation selon la revendication 10, pour améliorer la qualité de la peau.

12. Utilisation selon l'une des revendications 10 à 11, pour sublimer l'éclat du teint.

13. Utilisation selon l'une des revendications 10 à 12, pour rééquilibrer le microbiote des peaux matures.

14. Utilisation selon la précédente revendication, pour réduire la diversité bactérienne.

15. Utilisation selon la revendication 13 ou 14, pour diminuer l'abondance du phylum *Proteobacteria.*

16. Utilisation selon l'une des revendications 13 à 15, pour diminuer l'abondance du u genre *Corynebacterium.*

17. Utilisation selon l'une des revendications 10 à 16, pour renforcer la barrière mécanique de la peau.

18. Utilisation selon l'une des revendications 10 à 17, pour renforcer la barrière immune de la peau.

19. Utilisation selon la précédente revendication, pour stimuler la production des peptides antimicrobiens de la peau.

20. Utilisation selon la précédente revendication, pour stimuler la synthèse de la cohésion et de la différenciation des cellules cutanées et/ou pour réduire les pertes insensibles en eau de la peau.

21. Composition cosmétique comprenant au moins 0,5% en poids d'un principe actif selon l'une des revendications 1 à 9.

22. Composition cosmétique selon la précédente revendication, **caractérisée en ce qu'**elle se présente sous forme de crème ou gel.

23. Procédé cosmétique de traitement de la peau pour un effet d'amélioration et/ou de sublimation de l'éclat du teint, **caractérisé en ce qu'**il consiste en l'application topique sur la peau d'une composition selon l'une des revendications 21 ou 22.

24. Procédé cosmétique de traitement de la peau selon la revendication 23, pour un effet sur des peaux matures.

25. Procédé cosmétique de traitement de la peau selon l'une des revendications 23 ou 24, pour rééquilibrer le microbiote des peaux matures.

## Patentansprüche

1. Natürlicher kosmetischer Wirkstoff, umfassend mindestens einen Extrakt von *Metschnikowia reukaufii,* **dadurch gekennzeichnet, dass** der Extrakt ein Hydrolysat von *Metschnikowia reukaufii* ist.

2. Natürlicher kosmetischer Wirkstoff nach Anspruch 1, **dadurch gekennzeichnet, dass** der Extrakt von *Metschnikowia reukaufii* mindestens Peptide umfasst.

3. Natürlicher kosmetischer Wirkstoff nach dem vorstehenden Anspruch, **dadurch gekennzeichnet, dass** die Peptide mindestens 20 Gew.-% der Trockensubstanz des Extrakts, bestimmt durch die KJELDAHL-Methode, bilden.

4. Natürlicher kosmetischer Wirkstoff nach dem vorstehenden Anspruch, **dadurch gekennzeichnet, dass** die Peptide zwischen 20 und 60 Gew.-% der Trockensubstanz des Extrakts, bestimmt durch die KJELDAHL-Methode, bilden.

5. Natürlicher kosmetischer Wirkstoff nach einem der vorstehenden Ansprüche, **dadurch gekennzeichnet, dass** der Extrakt von *Metschnikowia reukaufii* Peptide umfasst, von denen mindestens 90 % eine Molekularmasse von weniger als 3500 Da aufweisen.

6. Natürlicher kosmetischer Wirkstoff nach einem der vorstehenden Ansprüche, **dadurch gekennzeichnet, dass** der Extrakt von *Metschnikowia reukaufii* Peptide umfasst, von denen mindestens 60 % eine Molekularmasse von weniger als 2000 Da aufweisen.

7. Natürlicher kosmetischer Wirkstoff nach einem der vorstehenden Ansprüche, **dadurch gekennzeichnet, dass** der Extrakt von *Metschnikowia reukaufii* auch Zucker umfasst.

8. Natürlicher kosmetischer Wirkstoff nach dem vorstehenden Anspruch, **dadurch gekennzeichnet, dass** die Zucker Glucose und Mannose in Form von Oligosacchariden mit einer mittleren Molekularmasse von weniger als 9000 Da umfassen.

9. Natürlicher kosmetischer Wirkstoff nach einem der vorstehenden Ansprüche, **dadurch gekennzeichnet, dass** der Extrakt von *Metschnikowia reukaufii* ein enzymatisches Hydrolysat von *Metschnikowia reukaufii* ist.

10. Verwendung eines natürlichen kosmetischen Wirkstoffs nach einem der Ansprüche 1 bis 9 oder einer diesen enthaltenden Zusammensetzung für eine kosmetische Behandlung bei topischer Anwendung auf der Haut.

11. Verwendung nach Anspruch 10 zum Verbessern der Hautqualität.

12. Verwendung nach einem der Ansprüche 10 bis 11 zum Sublimieren des Strahlens des Teints.

13. Verwendung nach einem der Ansprüche 10 bis 12 zum Ausgleichen der Mikrobiota reifer Haut.

14. Verwendung nach dem vorstehenden Anspruch zum Verringern der Bakterienvielfalt.

15. Verwendung nach Anspruch 13 oder 14 zum Verkleinern der Abundanz des Phylums *Proteobacteria.*

16. Verwendung nach einem der Ansprüche 13 bis 15 zum Verkleinern der Abundanz der Gattung *Corynebacterium.*

17. Verwendung nach einem der Ansprüche 10 bis 16 zum Verstärken der mechanischen Barriere der Haut.

18. Verwendung nach einem der Ansprüche 10 bis 17 zum Verstärken der Immunbarriere der Haut.

19. Verwendung nach dem vorstehenden Anspruch zum Stimulieren der Erzeugung von antimikrobiellen Peptiden der Haut.

20. Verwendung nach dem vorstehenden Anspruch zum Stimulieren der Synthese der Kohäsion und der Differenzierung der Hautzellen und/oder zum Verringern der unmerklichen Wasserverluste der Haut.

21. Kosmetische Zusammensetzung, umfassend mindestens 0,5 Gew.-% eines natürlichen Wirkstoffs nach einem der Ansprüche 1 bis 9.

22. Kosmetische Zusammensetzung nach dem vorstehenden Anspruch, **dadurch gekennzeichnet, dass** sie in Form einer Creme oder eines Gels vorliegt.

23. Kosmetisches Verfahren zum Behandeln der Haut für eine Verbesserungs- und/oder Sublimationswirkung des Strahlens des Teints, **dadurch gekennzeichnet, dass** es aus der topischen Anwendung einer Zusammensetzung auf der Haut nach einem der Ansprüche 21 oder 22 besteht.

24. Kosmetisches Verfahren zum Behandeln der Haut nach Anspruch 23 für eine Wirkung auf reife Haut.

25. Kosmetisches Verfahren zum Behandeln der Haut nach einem der Ansprüche 23 oder 24 zum Ausgleichen der Mikrobiota reifer Haut.

## Claims

1. Cosmetic active ingredient, comprising at least one *Metschnikowia reukaufii* extract, **characterized in that** the extract is a hydrolyzate of *Metschnikowia reukaufii.*

2. Cosmetic active ingredient according to claim 1, **characterized in that** the *Metschnikowia reukaufii* extract comprises at least peptides.

3. Cosmetic active ingredient according to the preceding claim, **characterized in that** the peptides represent at least 20 wt.% of dry matter of the extract, determined by the Kjeldahl method.

4. Cosmetic active ingredient according to the preceding claim, **characterized in that** the peptides represent between 20 and 60 wt.% of dry matter of the extract, determined by the Kjeldahl method.

5. Cosmetic active ingredient according to any of the preceding claims, **characterized in that** the *Metschnikowia reukaufii* extract comprises peptides of which at least 90% have a molecular mass of less than 3500 Da.

6. Cosmetic active ingredient according to any of the preceding claims, **characterized in that** the *Metschnikowia reukaufii* extract comprises peptides of which at least 60% have a molecular mass of less than 2000 Da.

7. Cosmetic active ingredient according to any of the preceding claims, **characterized in that** the *Metschnikowia reukaufii* extract also comprises sugars.

8. Cosmetic active ingredient according to the preceding claim, **characterized in that** the sugars comprise glucose and mannose in the form of oligosaccharides having an average molar mass of less than 9000 Da.

9. Active cosmetic ingredient according to any of the preceding claims, **characterized in that** the *Metschnikowia reukaufii* extract is an enzymatic *Metschnikowia reukaufii* hydrolyzate.

10. Use of a cosmetic active ingredient according to any of claims 1 to 9 or of a composition containing said active ingredient for a cosmetic treatment for topical application to the skin.

11. Use according to claim 10, for improving the quality of the skin.

12. Use according to either claim 10 or claim 11, for enhancing the radiance of the complexion.

13. Use according to any of claims 10 to 12, for rebalancing the microbiota of mature skin.

14. Use according to the preceding claim, for reducing bacterial diversity.

15. Use according to either claim 13 or claim 14, for decreasing the abundance of the phylum *Proteobacteria.*

16. Use according to any of claims 13 to 15, for reducing the abundance of the genus *Corynebacterium.*

17. Use according to any of claims 10 to 16, for strengthening the mechanical barrier of the skin.

18. Use according to any of claims 10 to 17, for strengthening the immune barrier of the skin.

19. Use according to the preceding claim, for stimulating the production of antimicrobial peptides in the skin.

20. Use according to the preceding claim, for stimulating the synthesis of cohesion and differentiation of skin cells and/or for reducing insensible water loss from the skin.

21. Cosmetic composition comprising at least 0.5 wt.% of an active ingredient according to any of claims 1 to 9.

22. Cosmetic composition according to the preceding claim, **characterized in that** it is in the form of a cream or gel.

23. Cosmetic method for treating the skin for an effect of improving and/or enhancing the radiance of the complexion, **characterized in that** said method consists of the topical application to the skin of a composition according to either claim 21 or claim 22.

24. Cosmetic method for treating the skin according to claim 23, for an effect on mature skin.

25. Cosmetic method for treating the skin according to either claim 23 or claim 24, for rebalancing the microbiota of mature skin.
